# EUROPEAN PATENT APPLICATION

(11) **EP 4 643 849 A1**
(43) Date of publication of application: **05.11.2025**
(21) Application number: 23911202.2
(22) Date of filing: 03.02.2023
(51) Int. Cl.: A61K 9/107, A23L 5/00, A23L 29/10, A61K 8/06

(54) **ACTIVE INGREDIENT-RETAINING AGENT**

(30) Priority: 28.12.2022 JP 2022211335
(71) Applicant: House Wellness Foods Corporation, Hyogo 664-0011 (JP); House Foods Corporation, Higashi-Osaka-shi, Osaka 577-8520 (JP)
(72) Inventor: TAGUCHI Hiromu, Higashi-osaka-city, Osaka 577-8520 (JP); TOMOTAKE Muneaki, Higashi-osaka-city, Osaka 577-8520 (JP); AOYAGI Morihiro, Higashi-osaka-city, Osaka 577-8520 (JP); ENOKIDA Takashi, Higashi-osaka-city, Osaka 577-8520 (JP)
(74) Representative: Hoffmann Eitle
(86) International application number: PCT/JP2023/003588
(87) International publication number: WO 2024/142417

(57) **Abstract**

An object of the present invention is to provide a novel method capable of enhancing and/or prolonging the effect of an active component. Provided is a retention agent to be used for retaining an active component, the retention agent including an oil-in-water emulsion containing water, a hydrophobic substance, cyclodextrin, and a thickening polysaccharide.

## Description

### Technical Field

The present invention relates to a retention agent to be used for retaining an active component, the retention agent including an oil-in-water emulsion containing water, a hydrophobic substance, cyclodextrin, and a thickening polysaccharide.

### Background Art

Today, emulsified compositions containing fat or oil, water, cyclodextrin, and a water-soluble gelling agent are widely used in various fields of, for example, pharmaceuticals, cosmetics, and foods or beverages.

Patent Literature 1 discloses an emulsified composition containing water, an oily component, α-cyclodextrin, and one or more of agar, low-strength agar, a combination of galactomannan or glucomannan and xanthan gum, carrageenan, furcelleran, gellan gum, and native gellan gum as gel-forming components, and also discloses pharmaceuticals, cosmetics, and chemical products using the emulsified composition.

Patent Literature 2 discloses an emulsified composition characterized by containing fat or oil, protein hydrolysate, water, cyclodextrin, and the like, and also discloses that gums such as xanthan gum, guar gum, gum arabic, CMC, carrageenan, and locust bean gum may be used as stabilizers.

Patent Literature 3 discloses an emulsified cosmetic obtained by mixing an aqueous raw material in which a water-soluble polymer compound such as pectin, tragacanth gum, gelatin, casein, sodium alginate, hydroxypropyl cellulose, hydroxyethyl cellulose, hydroxypropylmethyl cellulose, methyl cellulose, sodium polyacrylate, or carboxyvinyl polymer is dissolved with an oily raw material for a cosmetic, adding cyclodextrin thereto, and emulsifying the mixture.

Patent Literature 4 discloses an emulsified composition for skin containing water, fat or oil, cyclodextrin, and at least one water-soluble gelling agent selected from the group consisting of carboxymethyl cellulose (CMC), xanthan gum, locust bean gum, guar gum, glucomannan, κ-carrageenan, 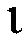 -carrageenan, λ-carrageenan tamarind gum, and gellan gum.

Non Patent Literature 1 describes that emulsion stability increased when xanthan gum and tragacanth gum were each added as an emulsion stabilizer to an oil-drops-in-water type emulsion obtained by emulsifying a sample of soybean oil and an aqueous cyclodextrin solution at a volume ratio of 1:1.

### Citation List

### Patent Literature

Patent Literature 1: Japanese Patent Laid-Open No. 2016-204311
Patent Literature 2: Japanese Patent Laid-Open No. 10-262560
Patent Literature 3: Japanese Patent Publication No. 61-038166
Patent Literature 4: WO2022/064997

### Non Patent Literature

Non Patent Literature 1: Nippon Shokuhin Kogyo Gakkaishi, Vol. 38, No. 1, 16-20 (1991)

### Summary of Invention

### Technical Problem

In the fields of, for example, medicines, cosmetics, and foods or beverages, it has long been desired to reduce the content of an active component, such as a drug or taste component, for reasons of reduced burden on subjects and increased health consciousness of consumers, and a method capable of enhancing and/or prolonging the effect of the active component has been thus required. Therefore, an object of the present invention is to provide a novel method capable of enhancing and/or prolonging the effect of an active component.

### Solution to Problem

As a result of intensive studies to solve the problem, the present inventors have found that an oil-in-water emulsion obtained by blending a hydrophobic substance, water, cyclodextrin, and a thickening polysaccharide could adhere to a tissue of an animal, hold an active component therein, and/or absorb/adsorb and hold the active component for retention, thereby enhancing and/or prolonging the effect of the active component in the tissue.

The present invention is based on these new findings, and the following inventions are included.
[1] A retention agent to be used for retaining an active component, the retention agent comprising an oil-in-water emulsion comprising water, a hydrophobic substance, cyclodextrin, and a thickening polysaccharide.
[2] The retention agent according to [1], wherein the hydrophobic substance is one or more selected from the group consisting of fat or oil, hydrophobic wax, and hydrophobic resin.
[3] The retention agent according to [1] or [2], wherein the thickening polysaccharide is one or more selected from the group consisting of carboxymethyl cellulose (CMC), xanthan gum, locust bean gum, guar gum, glucomannan, κ-carrageenan, 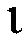 -carrageenan, λ-carrageenan, tamarind gum, gellan gum, gum arabic, pectin, distarch phosphate, hydroxypropyl starch, hydroxypropyl distarch phosphate, tragacanth gum, hydroxypropyl cellulose, methyl cellulose, hydroxypropylmethyl cellulose, and hydroxyethyl cellulose.
[4] The retention agent according to any one of [1] to [3], which is used by being added to a food or beverage.
[5] The retention agent according to any one of [1] to [3], which is used by being added to a medicine.
[6] The retention agent according to any one of [1] to [3], which is used by being added to a cosmetic.
[7] A method for producing a retention agent to be used for retaining an active component, the method comprising mixing water, a hydrophobic substance, cyclodextrin, and a thickening polysaccharide to form an oil-in-water emulsion.
[8] A food or beverage, comprising a retention agent to be used for retaining an active component, the retention agent comprising an oil-in-water emulsion comprising water, a hydrophobic substance, cyclodextrin, and a thickening polysaccharide, wherein the retention agent is contained in an amount of 0.5 mass% to 55 mass% in terms of an amount of the hydrophobic substance.
[9] A medicine, comprising a retention agent to be used for retaining an active component, the retention agent comprising an oil-in-water emulsion comprising water, a hydrophobic substance, cyclodextrin, and a thickening polysaccharide, wherein the retention agent is contained in an amount of 0.5 mass% to 55 mass% in terms of an amount of the hydrophobic substance.
[10] A cosmetic, comprising a retention agent to be used for retaining an active component, the retention agent comprising an oil-in-water emulsion comprising water, a hydrophobic substance, cyclodextrin, and a thickening polysaccharide, wherein the retention agent is contained in an amount of 0.5 mass% to 55 mass% in terms of an amount of the hydrophobic substance.
[11] Use of an oil-in-water emulsion comprising water, a hydrophobic substance, cyclodextrin and a thickening polysaccharide, in a method for producing a retention agent to be used for retaining an active component.

The present specification encompasses the contents described in, for example, the specifications of Japanese Patent Application No. 2022-211335, filed on December 28, 2022, which is the basis of the priority of the present application.

All publications, patents, and patent applications cited herein are hereby incorporated by reference in their entirety.

### Advantageous Effects of Invention

According to the present invention, it is possible to provide a retention agent for an active component that can retain the active component on the tissue of an animal and enhance and/or prolong the effect of the active component.

### Brief Description of Drawings

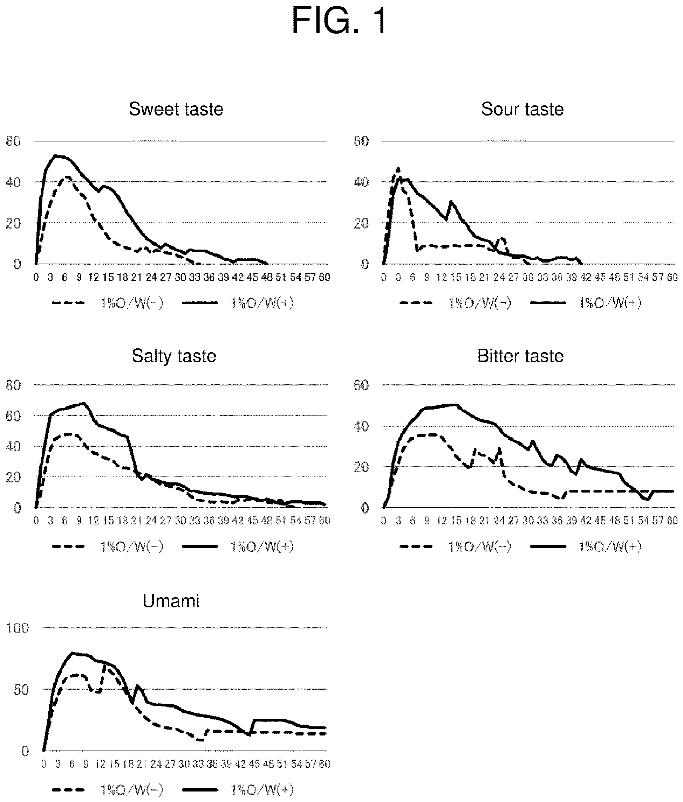

[Figure 1] Figure 1 is a graph showing results of evaluating changes in taste intensity for 1 minute by a time intensity (TI) method using sensory evaluation software FIZZ (Biosystemes Inc.) after ingestion of each sample prepared by blending components of each taste into an aqueous solution (1% O/W (+)) of an oil-in-water emulsion according to the present invention and an aqueous solution (1% O/W (-)) of a mixture (not forming an oil-in-water emulsion) of components constituting the oil-in-water emulsion (sweet taste: N = 4, salty taste and bitter taste: N = 5, sour taste and umami taste: N = 3. The results show average values),

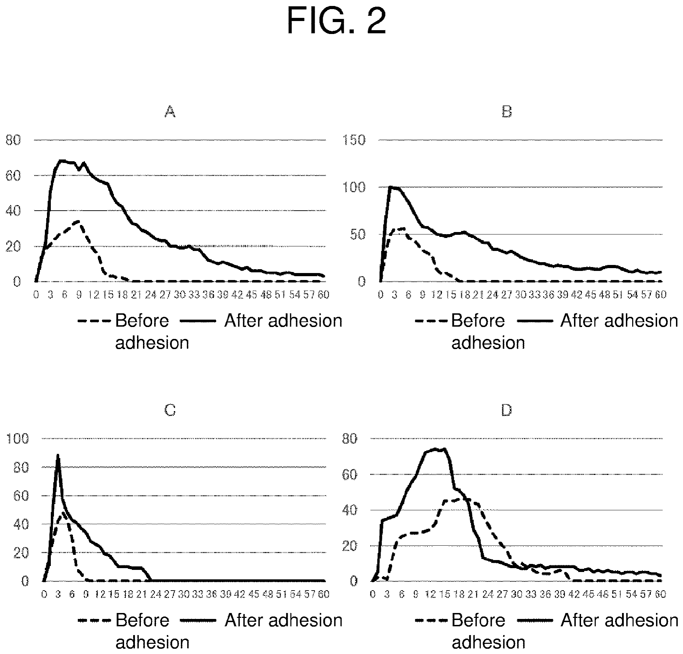

[Figure 2] Figure 2 is a graph showing results of evaluating changes in taste intensity for 1 minute by a time intensity (TI) method using sensory evaluation software FIZZ (Biosystemes Inc.) after ingestion of a saline solution before adhesion (Before adhesion) and after adhesion (After adhesion) of an aqueous solution (10% O/W (+)) of the oil-in-water emulsion according to the present invention to the oral cavity(N = 4).

### Description of Embodiments

### 1. Oil-in-Water Emulsion

The oil-in-water emulsion in the present invention contains water, a hydrophobic substance, cyclodextrin, and a thickening polysaccharide.

In the present invention, the "hydrophobic substance" may be any substance that is insoluble in water and capable of forming an oil-in-water emulsion together with water and cyclodextrin and thickening polysaccharides described in detail below. Examples of such hydrophobic substance preferably include, but are not limited to, fat or oil, hydrophobic wax, and hydrophobic resin. The hydrophobic substance may be used alone or in combination with different hydrophobic substances. For example, as the hydrophobic substance, one or more substances selected from the group consisting of fat or oil, hydrophobic wax, and hydrophobic resin can be used. An appropriate substance can be selected and used according to the utilization form of the retention agent of the present invention.

In the present invention, the "oil-in-water emulsion" means an emulsified composition in which a hydrophobic substance is dispersed in water, and cyclodextrin and thickening polysaccharides contribute to the formation and stability of this emulsified state.

As the "fat or oil" in the present invention, fat or oil commonly used in the formation of an oil-in-water emulsion is available, and both polar and non-polar fats or oils may be used. Examples of such fat or oil include, but are not limited to, plant-derived fat or oil (e.g., canola oil, refined rapeseed oil, soybean oil, corn oil, cottonseed oil, peanut oil, sesame oil, rice oil, rice bran oil, camellia oil, safflower oil, olive oil, linseed oil, Japanese basil oil, perilla oil, sunflower oil, palm oil, tea oil, coconut butter, avocado oil, *Aleurites moluccanus* seed oil, grapeseed oil, cocoa butter, coconut oil, wheatgerm oil, almond oil, evening primrose oil, castor oil, hazelnut oil, macadamia nut oil, rosehip oil, grape oil, cacao oil, jojoba oil, and palm kernel oil), synthetic ester oils such as isostearyl alcohol, caprylic alcohol, lauryl alcohol, stearyl alcohol, 2-octadecyl alcohol, myristyl alcohol, cetyl alcohol, phytosterol, cholesterol, stearic acid, isostearic acid, capric acid, lanolin acid, lauric acid, myristic acid, palmitic acid, behenic acid, linolic acid, linolenic acid, glyceryl monostearate, glyceryl monopalmitate, glyceryl monobehenate, glyceryl monomyristate, glyceryl monolaurate, glyceryl monolanolate, glyceryl monolinolate, glyceryl monolinolenate, glyceryl monooleate, glycerol triisostearate, isopropyl myristate, glycerol tri-2-heptylundecanoate, glycerol tri-2-ethylhexanoate, 2-heptylundecyl palmitate, di-2-heptylundecyl adipate, cetyl isooctanoate, trimethylolpropane-2-trimethylolheptylundecanoate, propane-2-ethylhexanoate, pentaerythritol-2-heptylundecanoate, pentaerythritol-2-ethylhexanoate, cholesterol isostearate, diethyl phthalate, and dibutyl phthalate, animal-derived fats or oils such as beef tallow, lard, lanolin, squalene, and squalane, and silicone oil. The fat or oil available in the present invention is preferably in a liquid state at least at normal temperature, preferably animal-derived or plant-derived fat or oil with high safety, and particularly preferably an edible vegetable oil that has been eaten and is highly safe. The fat or oil may be used alone or in combination with different fat or oil. An appropriate fat or oil can be selected and used according to the utilization form of the retention agent of the present invention. As used herein, the term "normal temperature" means 5 to 35°C, preferably 15 to 30°C.

In the present invention, the "hydrophobic wax" includes natural hydrophobic wax derived from animals and plants, petroleum, or minerals, and synthetic hydrophobic wax, and hydrophobic wax having a melting point of 80°C or lower can be used. The hydrophobic wax is preferably in a liquid state at least at normal temperature. Examples of such hydrophobic wax include, but are not limited to, beeswax, spermaceti, wool wax, Japan wax, rosin (pine resin), candelilla wax, carnauba wax, cocoa butter, paraffin wax, microcrystalline wax, ceresin wax, petroleum jelly wax, ozokenite wax, polyethylene wax, oxidized polyethylene wax, Fischer-Tropsch wax, alcohol-modified wax, maleic acid-modified oxidized polyethylene wax, and amide wax. In the present invention, the hydrophobic wax used is particularly preferably animal-derived or plant-derived hydrophobic wax with high safety. The hydrophobic wax may be used alone or in combination with different hydrophobic wax. An appropriate hydrophobic wax can be selected and used according to the utilization form of the retention agent of the present invention.

In the present invention, the "hydrophobic resin" refers to a resin that has no hydrophilic group or a small content thereof and is insoluble in a polar solvent such as water. In the present invention, a resin in a liquid state at least at normal temperature can be preferably used. Examples of such a hydrophobic resin include, but are not limited to, silicone resin (not hydrophilized), polyurethane resin, fluorine-containing resin, polyethylene resin, polypropylene resin, polyester resin, acrylic resin, polystyrene resin, polycarbonate resin, polyvinyl chloride resin, polysulfone resin, polyethersulfone resin, polyaramid resin, polyamide resin, polyether resin, polyacrylonitrile resin, polyetherimide resin, and copolymers of these polymers. The hydrophobic resin used in the present invention is particularly preferably a highly safe resin that has been confirmed to have biocompatibility and bioaffinity. The hydrophobic resin may be used alone or in combination with different hydrophobic resin. An appropriate hydrophobic resin can be selected and used according to the utilization form of the retention agent of the present invention.

In the present invention, the "hydrophobic substance" is preferably fat or oil and hydrophobic wax, particularly preferably fat or oil.

The oil-in-water emulsion of the present invention may contain a hydrophobic substance in any amount, for example, in an amount of 1 mass% or more, 3 mass% or more, 5 mass% or more, or 7 mass% or more. The upper limit thereof is not particularly limited, but may be, for example, 50 mass% or less, 40 mass% or less, 30 mass% or less, 20 mass% or less, or 10 mass% or less. The range of the amount of hydrophobic substance in the oil-in-water emulsion of the present invention can be expressed using two numerical values selected from each of the lower and upper numerical values. For example, the oil-in-water emulsion of the present invention may contain the hydrophobic substance in an amount appropriately selected from the range of 1 mass% to 50 mass%, 1 mass% to 30 mass%, 1 mass% to 20 mass%, or 1 mass% to 10 mass%, preferably 5 mass% to 10 mass%, or 7 mass% to 10 mass%. If the amount of the hydrophobic substance is less than 1 mass%, emulsification may be insufficient. On the other hand, if the amount of the hydrophobic substance is more than 50 mass%, the oil-in-water emulsion may give strong stickiness, sliminess, or the like. In either case, the oil-in-water emulsion may not be able to retain the active component or provide a desired feeling of use in the utilization form in the intended application.

In the present specification, the amount of each component contained in the oil-in-water emulsion of the present invention is expressed as an amount of mass%, where the total mass of the oil-in-water emulsion is 100 mass%.

In the oil-in-water emulsion of the present invention, water may be contained in any amount capable of emulsifying hydrophobic substance to form an oil-in-water emulsion together with cyclodextrin and thickening polysaccharide. For example, the oil-in-water emulsion of the present invention may contain water in an amount of 15 mass% or more, 20 mass% or more, 30 mass% or more, 40 mass% or more, 45 mass% or more, 50 mass% or more, 60 mass% or more, or 70 mass% or more. The upper limit thereof is not particularly limited, but may be, for example, 90 mass% or less, or 85 mass% or less. The range of the amount of the water in the oil-in-water emulsion of the present invention can be expressed using two numerical values selected from each of the lower and upper numerical values. For example, the oil-in-water emulsion of the present invention may contain water in an amount appropriately selected from the range of 15 mass% to 90 mass%, 45 mass% to 90 mass%, or 70 mass% to 85 mass%. The amount of water contained in the oil-in-water emulsion of the present invention is preferably more than that of hydrophobic substance in volume ratio. For example, the content of hydrophobic substance and water can be 1:greater than 1 (hydrophobic substance: water) in volume ratio, such as 1:2 or more, 1:3 or more, 1:4 or more, 1:5 or more, 1:6 or more, 1:10 or more, 1:15 or more, or 1:20 or more. The upper limit of the amount of water is not particularly limited, but can be 1:90 or less, 1:80 or less, 1:70 or less, 1:60 or less, or 1:50 or less. Through adjustment of the amounts of water and hydrophobic substance contained in the oil-in-water emulsion of the present invention within the above ranges, the oil-in-water emulsion may provide a feeling of use of suppressed stickiness, sliminess and the like.

"Cyclodextrin" means a cyclic non-reducing maltooligosaccharide having glucose as a constituent unit, and examples thereof include α-cyclodextrin having six glucose units, β-cyclodextrin having seven glucose units, and γ-cyclodextrin having eight glucose units. In the present invention, α-, β-, and γ-cyclodextrins, cyclodextrin derivatives, and any combination thereof can be used. Examples of the cyclodextrin derivatives include, but are not limited to, ethyl cyclodextrin, methyl cyclodextrin, hydroxyethyl cyclodextrin, hydroxypropyl cyclodextrin, methylamino cyclodextrin, amino cyclodextrin, carboxyethyl cyclodextrin, carboxymethyl cyclodextrin, sulfoxyethyl cyclodextrin, sulfoxyl cyclodextrin, acetyl cyclodextrin, branched cyclodextrin, cyclodextrin fatty acid ester, glucosyl cyclodextrin, and maltosyl cyclodextrin. Preferably, α-cyclodextrin is used. Since α-cyclodextrin has a high solubility in water, an oil-in-water emulsion with less roughness can be obtained.

In the oil-in-water emulsion of the present invention, cyclodextrin may be contained in an amount capable of contributing to emulsification and stability of the oil-in-water emulsion together with the thickening polysaccharide and providing a desired feeling of use in the utilization form in the intended application. For example, the oil-in-water emulsion of the present invention may contain cyclodextrin in an amount of 0.5 mass% or more, 1 mass% or more, 2.5 mass% or more, 3 mass% or more, 4 mass% or more, 4.5 mass% or more, or 5 mass% or more. The upper limit thereof is not particularly limited, but may be, for example, 15 mass% or less, 10 mass% or less, 9 mass% or less, 8 mass% or less, 7 mass% or less, or 6 mass% or less. The range of the amount of the cyclodextrin in the oil-in-water emulsion of the present invention can be expressed using two numerical values selected from each of the lower and upper numerical values. For example, the oil-in-water emulsion of the present invention may contain the cyclodextrin in an amount appropriately selected from the range of 0.5 mass% to 15 mass%, for example, 1 mass% to 15 mass%, 2.5 mass% to 10 mass%, 2.5 mass% to 9 mass%, 3 mass% to 9 mass%, 4 mass% to 8 mass%, 4.5 mass% to 9 mass%, or 5 mass% to 7 mass%, and preferably 2.5 mass% to 9 mass% or 4.5 mass% to 9 mass%. If the amount of the cyclodextrin is less than 0.5 mass%, the emulsification and stability of the oil-in-water emulsion may be insufficient. On the other hand, if the amount of the cyclodextrin is more than 15 mass%, the oil-in-water emulsion may have too high viscosity or give a strong creaky feeling. In either case, the oil-in-water emulsion may not be able to retain the active component or provide a desired feeling of use in the utilization form in the intended application.

In the present invention, the "thickening polysaccharide" generally means a polysaccharide that dissolves in water and imparts viscosity (sometimes also referred to as a thickening stabilizer or a water-soluble paste, for example). As the thickening polysaccharide available in the present invention, components usually used in the production of foods or beverages, medicines (including quasi-drugs), cosmetics, or the like can be used and are not particularly limited. The thickening polysaccharide may be used alone or in combination with different thickening polysaccharide. An appropriate thickening polysaccharide can be selected and used according to the utilization form of the retention agent of the present invention. Examples of the "thickening polysaccharide" available in the present invention preferably include carboxymethyl cellulose (CMC), xanthan gum, locust bean gum, guar gum, glucomannan, κ-carrageenan, 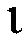 -carrageenan, λ-carrageenan, tamarind gum, gellan gum, gum arabic, pectin, distarch phosphate, hydroxypropyl starch, hydroxypropyl distarch phosphate, tragacanth gum, hydroxypropyl cellulose, methyl cellulose, hydroxypropylmethyl cellulose, and hydroxyethyl cellulose. Each of the thickening polysaccharides may be used alone or in combination with different thickening polysaccharides.

The "thickening polysaccharide" in the present invention is more preferably carboxymethyl cellulose (CMC), xanthan gum, locust bean gum, guar gum, glucomannan, K-carrageenan, 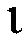 -carrageenan, λ-carrageenan, tamarind gum, or gellan gum, and even more preferably carboxymethyl cellulose (CMC), glucomannan, tamarind gum, or xanthan gum. These thickening polysaccharides can impart particularly high emulsion stability to the oil-in-water emulsion of the present invention.

In the oil-in-water emulsion of the present invention, the thickening polysaccharide may be contained in an amount capable of contributing to emulsification and stability of the oil-in-water emulsion together with cyclodextrin and providing a desired feeling of use in the utilization form in the intended application. For example, the oil-in-water emulsion of the present invention may contain a thickening polysaccharide in an amount of 0.05 mass% or more, 0.1 mass% or more, 0.2 mass% or more, or 0.3 mass% or more. The upper limit thereof is not particularly limited, but may be, for example, 1 mass% or less, 0.8 mass% or less, 0.7 mass% or less, 0.6 mass% or less, or 0.5 mass% or less. The range of the amount of the thickening polysaccharide in the oil-in-water emulsion of the present invention can be expressed using two numerical values selected from each of the lower and upper numerical values. For example, the oil-in-water emulsion of the present invention may contain the thickening polysaccharide in an amount appropriately selected from the range of 0.05 mass% to 1 mass%, 0.1 mass% to 1 mass%, 0.2 mass% to 0.8 mass%, or 0.2 mass% to 0.5 mass%. If the amount of the thickening polysaccharide is less than 0.05 mass%, the emulsification and stability of the oil-in-water emulsion may be insufficient. On the other hand, if the amount of the thickening polysaccharide is more than 1 mass%, the oil-in-water emulsion may have too high viscosity. In either case, the oil-in-water emulsion may not be able to retain the active component or provide a desired feeling of use in the utilization form in the intended application.

In the oil-in-water emulsion of the present invention, the cyclodextrin may be used in combination with the thickening polysaccharide to impart emulsification and stability of an oil-in-water emulsion. The emulsification and stability of the oil-in-water emulsion provided by the combined use of the cyclodextrin and the thickening polysaccharide do not depend on a three-dimensional matrix gel formed by dissolving a general gelling agent in water, followed by cooling (preferably, not including a three-dimensional matrix gel), but is considered to be due to the existence of an interaction by hydrogen bonds generated between the cyclodextrin and the thickening polysaccharide, as will be described in detail by the following Examples. Since the oil-in-water emulsion of the present invention has emulsification and stability by the combined use of the cyclodextrin and the thickening polysaccharide, the oil-in-water emulsion may be substantially free of an emulsifier, which is generally used in the production of a conventional emulsified composition, preferably substantially free of the emulsifier. In the present invention, the phrase "substantially free of an emulsifier" means that the oil-in-water emulsion of the present invention does not contain an emulsifier in such a manner as to exhibit an emulsifying effect, which is not intended to mean that the oil-in-water emulsion does not contain an emulsifier at all. The present invention can provide an advantageous oil-in-water emulsion with high safety, such as little irritation and low allergenicity, by being substantially free of an emulsifier, for example, when the oil-in-water emulsion of the present invention is applied. Examples of the "emulsifier generally used in the production of a conventional emulsified composition" include, but are not limited to, proteins, peptides or hydrolysates thereof, glycerin fatty acid esters, organic acid monoglycerides, polyglycerin fatty acid esters, polyoxyethylene glycerin fatty acid esters, sorbitan fatty acid esters, propylene glycol fatty acid esters, polyoxyethylene fatty acid esters, polyglycerin condensed ricinoleic acid esters, sorbitan fatty acid esters, polyoxyethylene sorbitol fatty acid esters, sucrose fatty acid esters, lecithin, enzymatically decomposed lecithin, polyethylene glycol, and polypropylene glycol.

The oil-in-water emulsion of the present invention is not limited to any particular form and can be provided in a form of a semi-solid/semi-liquid (e.g., a gel or a sol) or a dried body (e.g., a powder or a flake) prepared by reducing the water content, in addition to a form having the above-described water content. Such a form of a semi-solid/semi-liquid (e.g., a gel or a sol) or a dried body (e.g., a powder or a flake) can be formed after obtaining an oil-in-water emulsion in a form having the above water content by subjecting the oil-in-water emulsion to the conventionally known drying method to reduce the water content of the oil-in-water emulsion. Examples of such a drying method include, but are not limited to, freeze drying, heat drying, air drying, spray drying, drum drying, hot air drying, and vacuum drying. The water content of the oil-in-water emulsion after being subjected to the drying method can be appropriately selected depending on the intended form, and may be, for example, less than 15 mass%, 10 mass% or less, 5 mass% or less, or 1 mass% or less in the form of a dried body. The lower limit of the water content of the oil-in-water emulsion after being subjected to the drying method is not particularly limited but can be 0 mass% or more, more than 0 mass%, 0.1 mass% or more, 0.5 mass% or more, 0.6 mass% or more, 0.7 mass% or more, or 0.8 mass% or more. The range of the water content of the form of the dried body can be expressed using two numerical values selected from each of the lower and upper numerical values. For example, the water content of the oil-in-water emulsion may be 0 mass% to 15 mass%, more than 0 mass% and less than 15 mass%, 0.1 mass% to less than 15 mass%, 0.1 mass% to 10 mass%, 0.5 mass% to 5 mass%, 0.5 mass% to 1 mass%, 0.6 mass% to 1 mass%, 0.7 mass% to 1 mass%, or 0.8 mass% to 1 mass%. The oil-in-water emulsion of the present invention after being subjected to the drying method does not undergo a phase transition to yield a water-in-oil emulsion, and particularly in the form of a dried body, has high heat resistance and high moisture resistance without discoloration or dissolution even after being subjected to high temperature (e.g., about 100°C to 200°C) conditions and/or high humidity (e.g., about 95% humidity) conditions.

In addition to the above-described components, the oil-in-water emulsion of the present invention further contain, if necessary, components usually used in the production of the utilization form in the intended application, such as foods or beverages, medicines (including quasi-drugs), and cosmetic (hereinafter, referred to as "other components"), appropriately in an amount according to the desired utilization form as long as the effects of the present invention are not impaired. Examples of such other components include, but are not limited to, excipients, disintegrators, lubricants, binders, diluents, buffers, suspending agents, thickeners, preservatives, antibacterial agents, antiseptics, antioxidants, ultraviolet absorbers, coloring agents, pigments, dyes, colorants, lubricants, plasticizers, solvents, solubilizers, isotonic agents, correctives, vitamins, surfactants, pH adjusters, and chelating agents.

The oil-in-water emulsion of the present invention can be produced by mixing and stirring the water, hydrophobic substance, cyclodextrin, and thickening polysaccharide, as well as other components, if necessary, in the above-described amounts. All of the components may be mixed and stirred together, or each component may be added separately or sequentially in any combination (in any order), then mixed and stirred. The oil-in-water emulsion obtained by mixing and stirring may be subjected to a heat sterilization treatment.

The oil-in-water emulsion obtained by mixing and stirring may be further subjected to a drying method, if necessary. The drying method can be performed by the conventionally known drying method, and the water content of the oil-in-water emulsion can be appropriately adjusted according to the intended form of a semisolid/semi-liquid (e.g., gel or sol) or a dried body. The resulting dried body can be further subjected to crushing, grinding, or abrasion treatment, if necessary, to be in the form of a powder, a flake, or the like. The oil-in-water emulsion obtained by being subjected to the drying method may be mixed and stirred with other components in the above-described amounts, if necessary, and/or subjected to a heat sterilization treatment.

### 2. Application

The oil-in-water emulsion of the present invention can be used as a retention agent for retaining an active component.

In the present invention, the "retention agent" can retain an active component for a long time by holding or maintaining the active component at a predetermined site, thereby making it possible to enhance and/or maintain the effect of the active component for a long time as compared with the case where the retention agent is not used. When the retention agent of the present invention is administered or ingested with the active component to an animal, the oil-in-water emulsion adheres to the tissue at the application site, which holds the active component and/or absorbs/adsorbs and holds the active component to allow for retention, thereby enhancing and/ or maintaining the effect of the active component for a longer time than when the retention agent is not used.

The "animal" in the present invention is not particularly limited and may be any animal. The animal is preferably a mammal, and examples thereof include (but are not limited to) humans, domestic animals (e.g., cows, horses, sheep, pigs, goats), and pet animals (e.g., dogs, cats, rabbits, rabbits, hamsters, guinea pigs). The animal is more preferably a human.

In the present invention, examples of the "tissue" include various epithelial tissues found in the skin, oral cavity, esophagus, anus, vagina, nasal cavity, trachea, bronchus, stomach, small intestine, large intestine, oviduct, uterus, pleura, peritoneum, vascular endothelium, alveolus, thyroid gland (follicle), renal tubule, ureter, and bladder, hair, nail, and tooth. The "tissue" can be appropriately selected according to the intended application site. In the present invention, the "epithelial tissues" is preferably various epithelial tissues found in the skin, oral cavity, esophagus, nasal cavity, trachea, and bronchus, more preferably epithelial tissues in the oral cavity.

In the present invention, the phrase "administered or ingested together with an active component" means that the oil-in-water emulsion of the present invention and the active component may be administered or ingested within a time period in which both can coexist in the tissue at the application site. For example, the oil-in-water emulsion of the present invention and the active component may be contained in one composition and administered or ingested at the same time, or both may be administered or ingested in different forms and/or by different administration routes at the same time or before or after (preferably in the order of the oil-in-water emulsion of the present invention and then the active component). "Administration" only needs to be able to deliver the oil-in-water emulsion of the present invention and the active component to the tissue at the application site, which can be appropriately selected according to the form, amount, or the like of the oil-in-water emulsion of the present invention and the active component. Examples of the "administration" include, but are not limited to, oral administration, spray administration, aspiration, nasal administration, buccal administration, rectal administration, transdermal administration, and application. Preferably, the "administration" is oral administration, spray administration, aspiration, buccal administration, or application.

In the present invention, examples of the "active component" include a water-soluble component or a component having an affinity for the hydrophobic substance (e.g., a lipophilic component). The water-soluble component is considered to be held in the water of the oil-in-water emulsion of the present invention, preferably by a bond (e.g., a hydrogen bond) with the thickening polysaccharide and/or cyclodextrin. On the other hand, the component having an affinity for the hydrophobic substance is considered to be held in the hydrophobic substance in the oil-in-water emulsion of the present invention. Examples of the "active component" in the present invention include taste components in foods or beverages (salty taste components such as sodium chloride and potassium chloride; sweet taste components such as sugars (glucose, fructose, sucrose, maltose, oligosaccharide, isomerized sugar, etc.), sugar alcohols (xylitol, sorbitol, glycerin, erythritol, etc.), natural sweeteners (stevia, glycyrrhizin, thaumatin, etc.), and artificial sweeteners (aspartame, acesulfame potassium, sucralose, etc.); sour taste components such as citric acid, succinic acid, lactic acid, tartaric acid, acetic acid, fumaric acid, malic acid, gluconic acid, and ascorbic acid; umami taste components such as glutamic acid, inosinic acid, and guanylic acid; bitter taste components such as alkaloids (caffeine, quinine, strychnine, theobromine, etc.), terpenes (limonoid, limonin, obacunone, nomilin, cucurbitacin, humulone, lupron, etc.), glycosides (terpene glycoside, flavanone glycoside, naridine, neohesperidin, etc.), amino acids (tryptophan, phenylalanine, trypsin, arginine, valine, leucine, isoleucine, proline, etc.), casein, and soybean protein; pungent taste components such as capsaicin, piperine, sanshool, shogaol, gingerol, diallyl disulfide, p-hydroxybenzyl, and isothiocyanate; and astringent taste components such as tannin, catechin, theaflavin, and thearubigin (not limited thereto)), and food materials, seasonings, spices, and the like containing the same, and components used for predetermined treatments and purposes in medicines (including quasi-drugs) and cosmetics (hereinafter, referred to as "the medicines and the like") (e.g., drugs, bactericides, antimicrobial agents, antibiotics, enzymes, antibodies, vitamins, minerals, amino acids, refreshing agents, deodorants, moisturizers, and flavors (not limited thereto)).

The retention agent of the present invention can be used by being added to a food or beverage and the medicines and the like in order to retain an active component of the food or beverage and the medicines and the like. The amount of the retention agent added may be any amount as long as the retention agent can retain the active component of the food or beverage and the medicines and the like at a predetermined site for a longer period of time than when the retention agent is not used, and can enhance and/or prolong the effect of the active component, and can be appropriately determined depending on the type and amount of the active component, and the type and form of the food or beverage and the medicines and the like. For example, foods or beverages and the medicines and the like can contain the retention agent of the present invention in an amount of 0.1 mass% or more, 0.5 mass% or more, 1 mass% or more, 2.5 mass% or more, 5 mass% or more, or 7 mass% or more, in terms of the amount of the hydrophobic substance. The upper limit thereof is not particularly limited, but may be, for example, 55 mass% or less, 50 mass% or less, 40 mass% or less, 30 mass% or less, 20 mass% or less, or 10 mass% or less. The range of the amount of the retention agent of the present invention in foods or beverages and the medicines and the like can be expressed using two numerical values selected from each of the lower and upper numerical values. For example, foods or beverages and the medicines and the like may contain the retention agent of the present invention in an amount appropriately selected from the range of 0.5 mass% to 55 mass%, 0.5 mass% to 50 mass%, 0.5 mass% to 40 mass%, 0.5 mass% to 20 mass%, or 0.5 mass% to 10 mass%, in terms of the amount of the hydrophobic substance. When the amount of the retention agent of the present invention contained in foods or beverages and the medicines and the like is smaller or larger than the above range, the active component may not be retained in the utilization form in the intended application in either case.

In this specification, the amount of each component contained in foods or beverages and the medicines and the like is expressed as an amount of mass%, where the total mass of a solution of the foods or beverages and the medicines and the like, or the liquid portion of the foods or beverages and the medicines and the like excluding a solid portion (e.g., in the case of foods or beverages, the amount of the liquid portion of sauces, soups, or the like without ingredients) is 100 mass%. If the foods or beverages and the medicines and the like are in a liquid form or in a form without a liquid portion, the amount can be an amount in a solution prepared by adding an appropriate solvent (e.g., water, hot water, physiological saline, buffer) at the time of administration or ingestion or by being added to the appropriate solvent, or in a solution in which the foods or beverages and the medicines and the like are dissolved with water (e.g., a body fluid such as saliva) in the tissues of the application site.

The retention agent and active component of the present invention may be included together (e.g., in one composition) in foods or beverages and the medicines and the like. Alternatively, the retention agent and active component of the present invention may be in separate forms (e.g., each may be included in two or more compositions to be administered or ingested together).

The form of foods or beverages and the medicines and the like can be appropriately selected according to the utilization form in the intended application (e.g., the form of a predetermined product or the like), and can be taken in any form such as, but not limited to, solid, powder, granule, flake, chewable tablet, sheet, film, chewing gum, liquid, emulsion, semi-solid/semi-liquid (e.g., gel, sol, cream, paste, mousse), soft capsule, or the like, provided that the retention agent of the present invention is preferably present with the active component in the tissue at the application site in an amount that falls within the above range. Foods or beverages and the medicines and the like can be administered or ingested by adding an appropriate solvent (e.g., water, hot water, physiological saline, buffer), if necessary, or by adding an appropriate solvent and then adjusting the content of the retention agent of the present invention in these foods or beverages and the medicines and the like to within the above range, prior to administration or ingestion (examples of such a form of foods or beverages and the medicines and the like include, but are not limited to, a solid, a powder, a granule, a flake, a liquid, an emulsion, a semisolid/semi-liquid (e.g., a gel, a sol, a cream, a paste, a mousse)). Alternatively, foods or beverages and the medicines and the like may act by dissolving the retention agent of the present invention and the active component in the water content of the tissue at the application site (e.g., a body fluid such as saliva), with the content of the retention agent of the present invention adjusted to the above range (examples of such a form of foods or beverages and the medicines and the like include, but are not limited to, a powder, a granule, a chewable tablet, a sheet, a film, and a chewing gum).

In addition to the retention agent and the active component of the present invention, component usually used in the production of the utilization form in the intended application (e.g., the form of a predetermined product) can be appropriately blended into foods or beverages and the medicines and the like, if needed, in an amount according to the desired utilization form as long as the effects of the present invention are not impaired. Examples of such components include, but are not limited to, excipients, disintegrators, lubricants, binders, diluents, buffers, suspending agents, thickeners, preservatives, antibacterial agents, antiseptics, antioxidants, ultraviolet absorbers, coloring agents, pigments, dyes, colorants, lubricants, plasticizers, solvents, solubilizers, isotonic agents, correctives, perfumes, sweeteners, taste components, acidulants, seasonings, humectants, vitamins, surfactants, chelating agents, antibacterial agents, emulsifiers, water-soluble organic solvents, and food materials.

In one aspect, the retention agent of the present invention is provided in a food or beverage. Intake of the food or beverage allows the oil-in-water emulsion in the retention agent of the present invention to adhere to the epithelial tissue in the oral cavity, where the active component such as a taste component ingested together is held and/or absorbed/ adsorbed to hold the taste component for retention, thereby enhancing and/or prolonging the effect produced by the active component (for example, the flavors and umami brought by the taste component) as compared to when the retention agent is not used. The retention agent of the present invention in such utilization forms may also be referred to as a flavor enhancer, taste enhancer, or the like.

In another aspect, the "retention agent" of the present invention is provided in an oral care product. When the product is contained in the mouth, the oil-in-water emulsion in the retention agent of the present invention adheres to the epithelial tissue in the oral cavity, and a drug (e.g., a dental caries preventive/restorative agent (cetylpyridinium chloride (CPC), benzalkonium chloride, benzethonium chloride (BTC), sodium fluoride, sodium monofluorophosphate, etc.), a gingival/periodontal disease preventive/ameliorating agent (ε-aminocaproic acid, isopropylmethylphenol (cymen-5-ol), allantoin, dipotassium glycyrrhizinate, β-glycyrrhetinic acid, cetylpyridinium chloride (CPC), hinokitiol, triclosan, tranexamic acid, benzalkonium chloride, benzethonium chloride (BTC), triclosan, sodium chloride, tocopherol acetate, etc.), a prevention of mouth ulcer/ameliorating agent (allantoin, glycyrrhizic acid, tranexamic acid, etc.), a hypersensitivity preventive/inhibiting agent (aluminum lactate, potassium nitrate, etc.), a plaque preventive/removing agent (dextranase, etc.), a tartar preventive/removing agent (sodium polyphosphate, sodium pyrophosphate, etc.), a stain preventive/removing agent (sodium polyphosphate, polyethylene glycol, etc.), a halitosis preventive/removing agent (isopropylmethylphenol (cymen-5-ol), sodium lauroylsarcosine, etc.), a dry mouth preventive/inhibiting agent (hyaluronate, milk protein extract, lactoperoxidase, glucose oxidase, lysozymes, lactoferrins, potassium phosphate, dibasic, potassium chloride, calcium chloride hydrate, sodium chloride, magnesium chloride, pilocarpine hydrochloride, cevimeline hydrochloride hydrate, anetholtrithion, etc.), etc., but not limited thereto), which is an active component to be administered together with the retention agent, is held, and/or the drug is absorbed/adsorbed and held, thereby allowing the retention and enhancing and/or prolonging the effect of the drug compared with the case where the retention agent is not used. Such an intraoral care product can be in the conventional form known in the art, such as a mouthwash, a mouthrinse, a mouthwash, a liquid toothpaste, a toothpaste, a powdered toothpaste, a gel, a film, a chewing gum, a spray, and a liquid agent.

In another aspect, the "retention agent" of the present invention is provided in a hair care product. When the product is applied to the hair, the oil-in-water emulsion in the retention agent of the present invention adheres to the hair and holds the active components administered therewith, such as hair protective components (hydrolyzed eggshell membrane, hydrolyzed keratin, hydrolyzed collagen, hydrolyzed silk, hydrolyzed wheat, hydrolyzed wheat protein, hydrolyzed soybean protein, glutamate, arginine, pyrrolidone carboxylic acid, dimethicone, amodimethicone, polyquaternium-10, chitosans, etc.), hair texture improving components (stearyltrimonium-bromide, cetrimonium-bromide, stearyltrimonium-chloride, etc.), hair repair components (olive, jojoba, and avocado oils, squalane, isopropyl myristate, isostearyl alcohol, oleyl alcohol, yolk fat oil, adsorbed Purified Lanolin, soybean sterols, cholesterol, ceramides, etc.), and moisturizing components (hyaluronan, glycerin, hydrolyzed eggshell membrane, hydrolyzed keratin, hydrolyzed collagen, hydrolyzed silk, hydrolyzed wheat, hydrolyzed wheat protein, hydrolyzed soybean protein, glutamate, arginine, pyrrolidone carboxylic acid, yolk fat oil, adsorbed Purified Lanolin, soybean sterols, cholesterol, ceramides, etc.) (but not limited thereto) and/or absorbs and adsorbs to hold the active components, thereby allowing the retention and enhancing and/or prolonging the effect provided by the active components compared to the case where the retention agent is not used. Such a hair care product can be made in a conventionally known common form, such as shampoo, rinse, conditioner, and treatment.

Hereinafter, the present invention will be described with reference to Examples, but the present invention is not limited to these Examples.

### Examples

### Experiment 1: Evaluation of Emulsion Stability of Oil-in-water Emulsion

### (1) Preparation of oil-in-water emulsion

According to the composition in Table 1 below, each component was added and mixed to prepare an oil-in-water emulsion. Fat or oil (canola oil) was used as the hydrophobic substance. Carboxymethyl cellulose (CMC), glucomannan, guar gum, K-carrageenan, tamarind gum, gellan gum, xanthan gum, 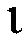 -carrageenan, locust bean gum, or λ-carrageenan was blended as the thickening polysaccharide, and any one of carboxyvinyl polymer and sodium polyacrylate, which are non-polysaccharide thickeners, was blended as a control.

Each component was mixed at once and stirred using a hand blender at 20,000 rpm for 10 minutes. Each oil-in-water emulsion (50 mL) obtained was transferred to a conical tube (Falcon (R) Conical Tube 50 mL) and centrifuged at 3,000 rpm for 1 minute. Subsequently, the thicknesses (depths) of water phase, micelles, and oil phase were visually observed, and the proportion of each phase (%) was measured, thereby evaluating the emulsion stability of each composition. The evaluation was made as "⊚" when the proportion of micelles was 100%, as "○" when the proportion of micelles was 70% or more and less than 100%, and as "×" when the proportion of micelles was less than 70%.

The amount of each component in the following table is expressed as an amount of mass%, where the amount of the obtained oil-in-water emulsion is 100 mass%. All of the fat or oil, α-cyclodextrin, and thickening polysaccharide used were food additives (food grade).

**[Table 1]**

| Component | Blending amount (mass%) |
|---|---|
| Hydrophobic substance: fat or oil (Canola oil + 1% oil red) | 25 |
| α-cyclodextrin | 2.5 |
| Thickening polysaccharide | 0.35 |
| Water | 72.15 |
| Total | 100 |

### (2) Result

The thickening polysaccharide added to the oil-in-water emulsion, the measured proportion of each phase, and the evaluation results are shown in Table 2. Although it is possible to form an oil-in-water emulsion by adding and mixing a thickening polysaccharide together with water, a hydrophobic substance, and α-cyclodextrin, it was confirmed that the micelle phase of the oil-in-water emulsion was maintained even under the above conditions and particularly high emulsion stability was exhibited when carboxymethyl cellulose (CMC), glucomannan, guar gum, K-carrageenan, tamarind gum, gellan gum, xanthan gum, 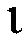 -carrageenan, locust bean gum, and λ-carrageenan were used as thickening polysaccharides, as is evident from the above results. In particular, when CMC, glucomannan, tamarind gum, and xanthan gum were each added, separation of the water phase and the oil phase was not observed while remarkably high emulsion stability was confirmed. On the other hand, the addition of a non-polysaccharide thickener resulted in a large separation between the water phase and the oil phase under the above conditions, and it was confirmed that the emulsion stability was relatively low.

**[Table 2]**

| Thickening polysaccharide added (Control: non-polysaccharide thickener) | Proportion of each phase (%) | | | Emulsion stability |
|---|---|---|---|---|
| | Water phase | Micelle | Oil phase | |
| None | 0.00% | 70.27% | 29.73% | - |
| Carboxymethyl cellulose (CMC) | 0.00% | 100.00% | 0.00% | ⊚ |
| Glucomannan | 0.00% | 100.00% | 0.00% | ⊚ |
| Guar gum | 0.00% | 91.11% | 8.89% | ○ |
| κ-carrageenan | 6.67% | 93.33% | 0.00% | ○ |
| Tamarind gum | 0.00% | 100.00% | 0.00% | ⊚ |
| Gellan gum | 8.11% | 91.89% | 0.00% | ○ |
| Xanthan gum | 0.00% | 100.00% | 0.00% | ⊚ |
| 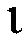 -carrageenan | 2.27% | 97.73% | 0.00% | ○ |
| Locust bean gum | 0.00% | 97.06% | 2.94% | ○ |
| λ-carrageenan | 4.44% | 95.56% | 0.00% | ○ |
| Carboxylvinyl polymer | 28.50% | 28.50% | 43.00% | × |
| Sodium polyacrylate | 0.00% | 60.00% | 40.00% | × |

According to the composition in Table 3 below, each component was added and mixed to prepare an oil-in-water emulsion. Any one of carnauba wax, petroleum jelly wax (white vaseline), rosin, and silicone oil containing oil red in an amount of 1% was used as the hydrophobic substance, and xanthan gum was blended as the thickening polysaccharide. The amount of each component in the table is expressed as an amount of mass%, where the amount of the obtained oil-in-water emulsion is 100 mass%.

Each component was mixed and stirred in the same manner as described above, and each oil-in-water emulsion (50 mL) obtained was transferred to a conical tube (Falcon (R) Conical Tube 50 mL) and centrifuged at 3,000 rpm for 1 minute. Subsequently, the thicknesses (depths) of water phase, micelles, and oil phase were visually observed, and the proportion of each phase (%) was measured, thereby evaluating the emulsion stability of each composition in the same manner as described above.

**[Table 3]**

| Component | Blending amount (mass%) |
|---|---|
| Hydrophobic substance: (Hydrophobic substance + 1% oil red) | 25 |
| α-cyclodextrin | 2.5 |
| Thickening polysaccharide (Xanthan gum) | 0.35 |
| Water | 72.15 |
| Total | 100 |

The hydrophobic substance added to the oil-in-water emulsion, the measured proportion of each phase, and the evaluation results are shown in Table 4. From the results, it was confirmed that an oil-in-water emulsion can be formed even when not only fat or oil but also other hydrophobic substances are used, and that the micelle phase of the oil-in-water emulsion is maintained even under the above conditions, resulting in high emulsion stability.

**[Table 4]**

| Hydrophobic substance added | Proportion of each phase (%) | | | Emulsion stability |
|---|---|---|---|---|
| | Water phase | Micelle | Oil phase | |
| Carnauba wax | 0.00% | 100.00% | 0.00% | ⊚ |
| Petroleum jelly wax | 0.00% | 100.00% | 0.00% | ⊚ |
| Rosin | 0.00% | 100.00% | 0.00% | ⊚ |
| Silicone oil | 0.00% | 100.00% | 0.00% | ⊚ |

### Experiment 2: Evaluation of Interaction between α-Cyclodextrin and Thickening Polysaccharide in Oil-in-Water Emulsion

To evaluate the interaction between α-cyclodextrin and thickening polysaccharide in an aqueous solution, enthalpy change (ΔH) was determined by isothermal titration calorimetry (ITC) in accordance with a conventionally known method. Specifically, using an isothermal titration calorimeter (NANO ITC SV; TA Instruments), 10 µL of an aqueous solution of thickening polysaccharide (0.05 g/100 mL) was added dropwise to an aqueous solution of α-cyclodextrin(25 g/100 mL) 25 times at intervals of 180 seconds (75 minutes) to determine an enthalpy value (µJ) from the area of the titration peak at the 25th (final) titration. The enthalpy value (µJ) was determined in the same manner using a non-polysaccharide thickener such as carboxylvinyl polymer or sodium polyacrylate, as a control.

The results are shown in Table 5. Positive enthalpy values, i.e., endothermic reactions, were observed when the thickening polysaccharide (CMC and xanthan gum), which had particularly high emulsion stability in Experiment 1, were used. This result suggests the presence of an interaction between the thickening polysaccharide and α-cyclodextrin via hydration water, and it is considered that, for example, a part of water molecules hydrated in the thickening polysaccharide forms a hydrogen bond with α-cyclodextrin. A hydrogen bond formed between water molecules usually has a shorter distance and a higher energy value than those of a hydrogen bond formed between the thickening polysaccharide and α-cyclodextrin, which causes intermolecular repulsion due to a charged site. Therefore, when a part of water molecules hydrated in the thickening polysaccharide forms a hydrogen bond with α-cyclodextrin, it is considered that an endothermic reaction occurs as a result of the subtraction of energy values. The heat transfer (positive enthalpy value) suggests the presence of the interaction between the thickening polysaccharide and α-cyclodextrin, and this interaction is considered to be a factor in giving the oil-in-water emulsion high emulsion stability in Experiment 1.

**[Table 5]**

| Thickening polysaccharide (Control: non-polysaccharide thickener) | Enthalpy value (µJ) |
|---|---|
| Carboxymethyl cellulose (CMC) | 247.1 |
| Xanthan gum | 326.1 |
| Carboxylvinyl polymer | -23.53 |
| Sodium polyacrylate | -22.36 |
| Water-water | -19.23 |

### Experiment 3 Performance Evaluation of Oil-in-Water Emulsions (Flavor Enhancement)

### (1) Flavor Evaluation of Oil-in-Water Emulsion Itself

According to the composition in Table 6 below, each component was added and mixed to prepare an oil-in-water emulsion. Each component was mixed at once and stirred using a hand blender at 20,000 rpm for 10 minutes.

The amount of each component in the table is expressed as an amount of mass%, where the amount of the obtained oil-in-water emulsion is 100 mass%.

A mixture prepared by mixing components other than fat or oil according to the composition in Table 6 below, then adding the fat or oil, and mixing the mixture by hand without applying a shear force (i.e., an oil-in-water emulsion was not prepared) was used as a control.

The resulting oil-in-water emulsion and the control mixture were each added to water in an amount of 1 mass%, and the presence or absence of flavor of each solution was subjected to sensory evaluation by well-trained professional panelists.

**[Table 6]**

| Component | Blending amount (mass%) |
|---|---|
| Hydrophobic substance: fat or oil (palm oil) | 13 |
| α-cyclodextrin | 8 |
| Thickening polysaccharide (Xanthan gum) | 0.3 |
| Water | 78.7 |
| Total | 100 |

As a result, both the oil-in-water emulsion and the control mixture had no difference in smell compared to plain water.

The control mixture, when ingested, gave a slight slimy sensation derived from fat or oil as compared with water, but no difference in taste.

The oil-in-water emulsion, when ingested, gave the entire oral cavity was widely covered (the oil-in-water emulsion was attached) as compared with water, but no difference in taste. It was confirmed that the amount of the fat or oil contained in the 1 mass% oil-in-water emulsion solution was only about 0.13 mass%, and no taste or smell derived from the fat or oil could be detected.

### (2) Effect of Combined Use of Oil-in-Water Emulsion on Flavor Enhancement I

To each of the 1 mass% oil-in-water emulsion solution and the 1 mass% control mixture solution obtained above was added each taste component shown in Table 7 below in a predetermined amount to prepare a sample for flavor evaluation (hereinafter, a sample containing the 1 mass% oil-in-water emulsion solution is referred to as "1% O/W(+)", and a sample containing the 1 mass% control mixture solution is referred to as "1% O/W(-)"). As a control, an aqueous solution (hereinafter, referred to as "control aqueous solution") obtained by adding the same amount of only the component of each taste to water was further used.

In the evaluation order given in Table 7 below, well-trained professional panelists ingested each sample (10 mL) and evaluated the intensity of each taste on a 10-point scale, with a rating of 5 for the control solution. Rinsing was performed twice with 20 mL of water.

**[Table 7]**

| | |
|---|---|
| Component of taste and amount | Sweet taste: 1.6 mass% of sucrose |
| | Salty taste: 0.6 mass% of salt |
| | Umami: 0.3 mass% of sodium glutamate |
| | Sour taste: 0.1 mass% of citric acid |
| | Bitter taste: 0.1 mass% of caffeine |
| Order of evaluation | Control aqueous solution → rinsing → 1% O/W (-)→ rinsing → 1% O/W (+) |

The evaluation results of the respective taste intensity of each sample are shown in Table 8 below (sweet taste, umami, sour taste, and bitter taste: N = 4, salty taste: N = 3).

**[Table 8]**

| | Taste intensity |
|---|---|
| | Control aqueous solution: 1 % O/W (-): 1% O/W (+) |
| Sweet taste | 5:6:9, 5:6:8, 5:7:9, 5:6:9 |
| Salty taste | 5:6:8, 5:6:7, 5:6:8 |
| Umami | 5:6:7, 5:6:7, 5:6:10, 5:5:7 |
| Sour taste | 5:6:8, 5:6:8, 5:5:8, 5:6:7 |
| Bitter taste | 5:5:9, 5:5:9, 5:6:10, 5:7:8 |

From the above results, it was confirmed that the inclusion of components of taste in the oil-in-water emulsion (1% O/ W (+)) significantly enhanced the taste intensity. When the taste component was included in a mixture that did not form an oil-in-water emulsion (1% O/W (-)), there was also a tendency for the taste intensity to increase slightly, the extent of which was insignificant.

Furthermore, well-trained professional panelists evaluated changes in taste intensity of 1% O/W(+) and 1% O/W(-) for 1 minute after ingestion of each sample by the time intensity (TI) method using the sensory evaluation software FIZZ (Biosystemes Inc.). The results are shown in Figure 1 (sweet taste: N = 4, salty taste and bitter taste: N = 5, sour taste and umami: N = 3. The results in Figure 1 show the average value of the results of each panelist).

From the results of Figure 1, it was confirmed that 1% O/W (+) showed higher values in one or more of rise immediately after intake, intensity, middle thickness, and persistence of aftertaste for each taste than 1% O/W(-).

### (3) Effect of Combined Use of Oil-in-Water Emulsion on Flavor Enhancement II

According to the composition in Table 9 below, each component was added and mixed to prepare an oil-in-water emulsion. As the thickening polysaccharide, xanthan gum, gum arabic, pectin, distarch phosphate, hydroxypropyl starch, hydroxypropyl distarch phosphate, tragacanth gum, hydroxypropyl cellulose, methyl cellulose, hydroxypropylmethyl cellulose, or hydroxyethyl cellulose was used. Each component was mixed at once and stirred using a hand blender at 20,000 rpm for 10 minutes.

The amount of each component in the table is expressed as an amount of mass%, where the amount of the obtained oil-in-water emulsion is 100 mass%.

A mixture prepared by mixing components other than fat or oil according to the composition in Table 9 below, then adding the fat or oil, and mixing the mixture by hand without applying a shear force (i.e., an oil-in-water emulsion was not prepared) was used as a control.

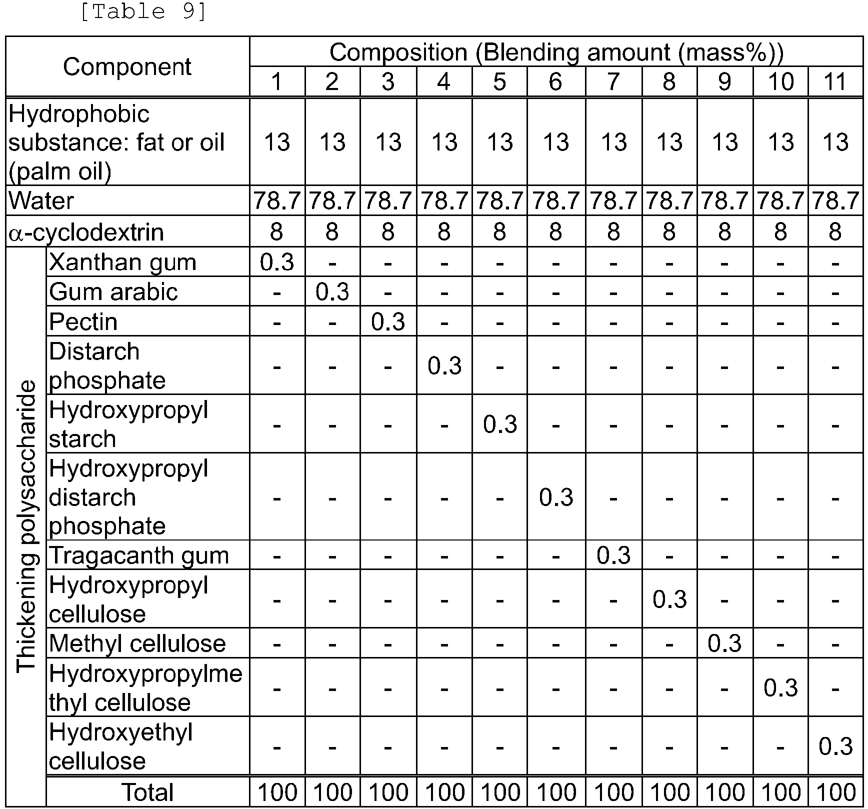

The resulting oil-in-water emulsion and the control mixture were each added to water in an amount of 1 mass% to prepare a 1 mass% oil-in-water emulsion solution and 1 mass% control mixture solution. Salt was added to each solution in an amount of 0.6 mass% as a sample for flavor evaluation (hereinafter, the sample containing the 1 mass% oil-in-water emulsion solution is referred to as "1% O/W (+)", and the sample containing the 1 mass% control mixture solution is referred to as "1% O/W (-)"). As a control aqueous solution, an aqueous solution of salt added to water in an amount of 0.6 mass% was further used.

In the evaluation order given in Table 7 above, well-trained professional panelists ingested each sample (10 mL) and evaluated the intensity of each taste on a 10-point scale, with a rating of 5 for the control solution. Rinsing was performed twice with 20 mL of water.

The evaluation results of the respective taste intensity of each sample are shown in Table 10 below (N= 3).

**[Table 10]**

| Composition | Taste intensity |
|---|---|
| | Control aqueous solution: 1% O/W (-):1% O/W (+) |
| 1 | 5:6:8, 5:6:7, 5:6:8 |
| 2 | 5:6:8, 5:6:7, 5:5:9 |
| 3 | 5:6:8, 5:6:8, 5:6:9 |
| 4 | 5:5:7, 5:5:7, 5:5:7 |
| 5 | 5:5:7, 5:5:7, 5:5:7 |
| 6 | 5:5:7, 5:5:7, 5:5:7 |
| 7 | 5:6:8, 5:6:8, 5:5:8 |
| 8 | 5:6:8, 5:6:8, 5:6:8 |
| 9 | 5:5:9, 5:6:8, 5:5:9 |
| 10 | 5:6:9, 5:6:9, 5:5:9 |
| 11 | 5:5:9, 5:6:8, 5:5:9 |

From the above results, it was confirmed that the inclusion of the oil-in-water emulsion (1% O/ W (+)) significantly enhanced the taste intensity, regardless of the type of thickening polysaccharide used.

### (4) Effect of Combined Use and Repeated Ingestion of Oil-in-Water Emulsion on Flavor Enhancement

A 1% O/W (+) containing a taste component (0.6 mass% of salt or 0.2 mass% of caffeine) prepared in the same manner as described in (2) above was ingested (10 mL each) four times consecutively (without rinsing) to evaluate the intensity of each taste on a 10-point scale, with a rating of 5 for the first ingestion. The evaluation was made by well-trained professional panelists.

The evaluation results of the respective taste intensity of each sample are shown in Table 11 below (salty taste: N = 4, bitter taste: N= 3).

**[Table 11]**

| | Taste intensity |
|---|---|
| | 1st → 2nd → 3rd → 4th |
| Salty taste | 5→6→6.5→6.5 |
| | 5→5.5→5.5→5 |
| | 5→5→6→7 |
| | 5→6→6.5→6.5 |
| Bitter taste | 5→6→7→9 |
| | 5→7→7→8 |
| | 5→6→7→8 |

From the above results, it was confirmed that the intensity of taste increased stepwise with repeated ingestion of samples containing an oil-in-water emulsion in any case, which may be due to accumulation of taste components adhering to the oral cavity together with the oil-in-water emulsion.

### (5) Effect of Separate Use of Oil-in-Water Emulsion on Flavor Enhancement I

Each of the oil-in-water emulsions prepared in (1) above was added to water in an amount of 10 mass% to obtain a 10 mass% aqueous solution of the oil-in-water emulsion containing no taste components (hereinafter referred to as "10% O/W (+)"). A 0.6 mass% saline solution was used as the aqueous solution to which the taste components were added (hereinafter referred to as an "aqueous taste solution").

In the evaluation, an aqueous taste solution (10 mL) was ingested, the taste intensity was evaluated (before adhesion), the mouth was washed (rinsed with 20 mL of water twice), then 10% O/W (+) (10 mL) was contained in the mouth for 30 seconds, adhered to the oral cavity, and then spit out. The mouth was washed (rinsed with 20 mL of water twice), and then the aqueous taste solution (10 mL) was ingested again to evaluate the taste intensity (after adhesion). The evaluation was made by well-trained professional panelists on a 10-point scale, with a rating of 5 for aqueous taste solutions before the adhesion.

The evaluation results obtained are shown in Table 12 below (N = 4).

**[Table 12]**

| | Taste intensity | |
|---|---|---|
| | before adhesion of Oil-in-water emulsion | after adhesion of Oil-in-water emulsion |
| Salty taste | 5 | 8 |
| | 5 | 7 |
| | 5 | 8 |
| | 5 | 7 |

Furthermore, well-trained professional panelists evaluated changes in taste intensity of an aqueous taste solution before and after adhesion of an oil-in-water emulsion for 1 minute after ingestion of each sample by the time intensity (TI) method using the sensory evaluation software FIZZ (Biosystemes Inc.). The results are shown in Figure 2 (N=4).

From the results, it was confirmed that taste intensity was enhanced even by ingesting the aqueous taste solution after the oil-in-water emulsion was adhered to the oral cavity without mixing the taste components in an aqueous solution of the oil-in-water emulsion in advance. In addition, as shown in Figure 2, it was confirmed that when the aqueous taste solution was ingested after the oil-in-water emulsion was adhered, high values were obtained for the initial rise immediately after intake, intensity, and thickness of middle of taste, and persistence of aftertaste, presumably because the taste component adhered to and accumulated in the oil-in-water emulsion, to which the taste component had adhered, and acted thereon.

### (6) Effect of Separate Use of Oil-in-Water Emulsion on Flavor Enhancement II

An aqueous taste solution was prepared by adding the components of each taste shown in Table 13 below to water in predetermined amounts, and the intensity of each taste of the aqueous taste solution before and after adhesion of 10% O/W (+) to the oral cavity was evaluated in the same manner as in the evaluation method described in (5) above. The evaluation was made by well-trained professional panelists on a 10-point scale, with a rating of 5 for each aqueous taste solution before the adhesion.

The evaluation results obtained are shown in Table 13 below (N = 2).

**[Table 13]**

| | |
|---|---|
| Component of taste and amount | Sour taste: 0.2 mass% of citric acid |
| | Salty taste: 1.2 mass% of salt |
| | Sweet taste: 3.2 mass% of sucrose |
| | Umami: 0.6 mass% of sodium glutamate |
| | Bitter taste: 0.2 mass% of caffeine |

| | Taste intensity | |
|---|---|---|
| | before adhesion of Oil-in-water emulsion | after adhesion of Oil-in-water emulsion |
| Sour taste | 5 | 8 |
| | 5 | 9 |
| Salty taste | 5 | 7 |
| | 5 | 8 |
| Sweet taste | 5 | 7 |
| | 5 | 8 |
| Umami | 5 | 7 |
| | 5 | 7 |
| Bitter taste | 5 | 8 |
| | 5 | 9 |

From the above results, it was confirmed that, for all five basic tastes, taste intensity was enhanced by simply ingesting the aqueous taste solution after the oil-in-water emulsion was adhered to the oral cavity without mixing the taste components in an aqueous solution of the oil-in-water emulsion in advance.

### (7) Effect of Combined Use of Oil-in-Water Emulsion on Flavor Enhancement of Pungent Component

All of the five basic tastes are sensed by the gustatory nerve. On the other hand, pungent and astringent tastes are sensed not by the gustatory nerve but by the sense of pain and temperature, and are thus distinguished from the above-described taste components. Therefore, it was confirmed whether the combination of oil-in-water emulsions can produce the same effect on components different from the taste components.

According to the composition in Table 14 below, each component was added and mixed to prepare an oil-in-water emulsion containing a pungent component in the fat or oil. As the thickening polysaccharide, xanthan gum, gum arabic, pectin, distarch phosphate, hydroxypropyl starch, hydroxypropyl distarch phosphate, tragacanth gum, hydroxypropyl cellulose, methyl cellulose, hydroxypropylmethyl cellulose, or hydroxyethyl cellulose was used. Each component was mixed at once and stirred using a polytron homogenizer at 20,000 rpm for 1 minute.

The amount of each component in the table is expressed as an amount of mass%, where the amount of the obtained oil-in-water emulsion is 100 mass%.

As a control, chili oil was used.

For evaluation, well-trained professional panelists ingested each sample (10 mL) and rated each taste intensity on a 10-point scale, with a control rating of 5. The results are shown in Table 14, where each numerical value represents the average value (N = 3).

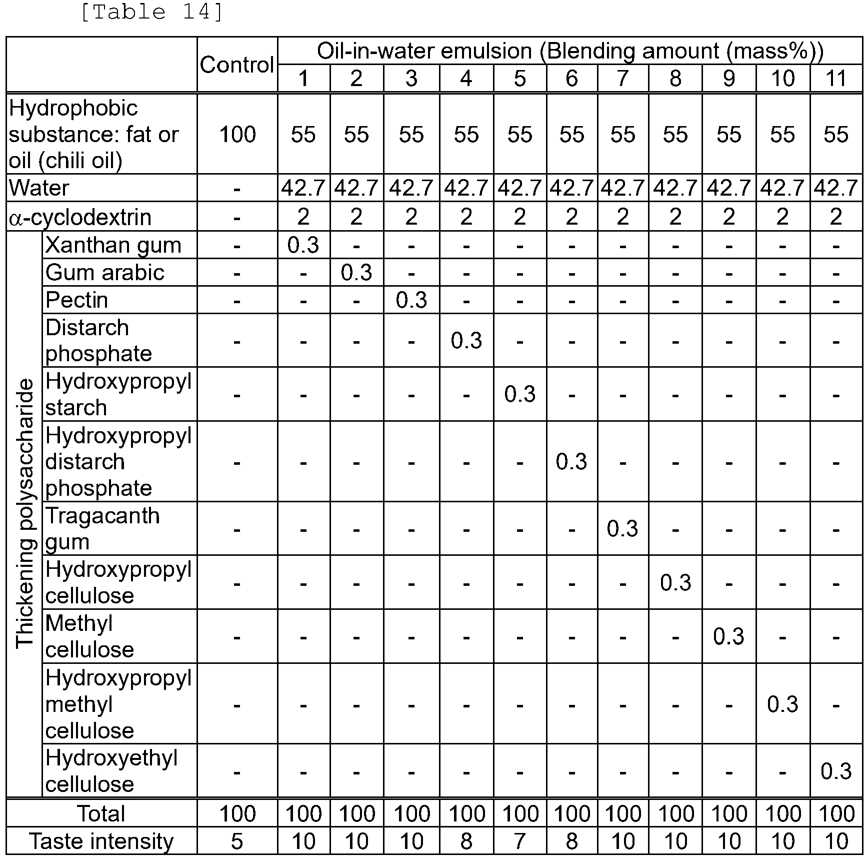

As a result, it was confirmed that all oil-in-water emulsions containing a pungent component had a higher intensity of pungent taste than the control, regardless of the type of thickening polysaccharide used, and that the oil-in-water emulsions containing a pungent component continued to be pungent for 10 minutes or more after ingestion, while the pungent taste after ingestion of the control was no longer felt in about 2 minutes.

From these results, it was confirmed that the effect (intensity) and durability can be enhanced by using oil-in-water emulsions in combination with components other than the taste components, or components in fat or oil.

### (8) Comparative Example

According to the composition in Table 15 below, each component was added and mixed to prepare an emulsified composition. Each component was mixed and stirred using a polytron homogenizer at 20,000 rpm for 1 minute.

The evaluation was conducted by dividing into a set of Comparative Examples A and Comparative Examples B to confirm whether the emulsified compositions (A-2 and B-2) had a stronger salty taste than the emulsified compositions (A-1 and B-1) without α-cyclodextrin and thickening polysaccharide (xanthan gum), respectively.

For evaluation, well-trained professional panelists (N = 3) ingested each emulsified composition (A-1 and B-1) (10 mL), evaluated the taste intensity, rinsed the mouth (rinsed with 20 mL water twice), and then ingested each emulsified composition (A-2 and B-2), evaluated and compared the taste intensity.

**[Table 15]**

| | Comparative Example A | | Comparative Example B | |
|---|---|---|---|---|
| | Comparative Example A-1 | Comparative Example A-2 | Comparative Example B-1 | Comparative Example B-2 |
| Component | Blending amount (mass%) | | Blending amount (mass%) | |
| Hydrophobic substance: fat or oil (palm oil) | 90.5 | 90.5 | 56.5 | 56.5 |
| α-cyclodextrin | 0 | 0.5 | 0 | 0.5 |
| Thickening polysaccharide (Xanthan gum) | 0 | 0.1 | 0 | 0.1 |
| Emulsifier (glycerin fatty acid ester) | 2.5 | 2.5 | 2.5 | 2.5 |
| Salt | 1 | 1 | 1 | 1 |
| Water | 6 | 5.4 | 40 | 39.4 |
| Total | 100 | 100 | 100 | 100 |

In Comparative Example A, both Comparative Example A-1 and Comparative Example A-2 had a butter-like shape and had almost no salty taste. Comparing Comparative Example A-1 and Comparative Example A-2, there was no difference in the intensity of salty taste between the two.

In Comparative Example B, emulsification was insufficient, and oil floated in both Comparative Example B-1 and Comparative Example B-2. Both Comparative Example B-1 and Comparative Example B-2 had a salty taste but no residual taste, and there was no difference in the intensity of the salty taste between the two.

### Experiment 4: Performance Evaluation of Oil-in-Water Emulsions (Enhancement of Hair Care Effect)

According to the composition in Table 16 below, each component was added and mixed to prepare an oil-in-water emulsion. Each component was mixed at once and stirred using a hand blender at 20,000 rpm for 10 minutes. The amount of each component in the table is expressed as an amount of mass%, where the amount of the obtained oil-in-water emulsion is 100 mass%.

**[Table 16]**

| Component | Blending amount (mass%) |
|---|---|
| Hydrophobic substance: fat or oil (palm oil) | 13 |
| α-cyclodextrin | 8 |
| Thickening polysaccharide (Xanthan gum) | 0.3 |
| Water | 78.7 |
| Total | 100 |

The resulting oil-in-water emulsion was added to a commercially available hair conditioner (TSUBAKI Premium Moist Hair Conditioner, (FineToday Holdings Co., Ltd)) in an amount of 25% by mass to prepare an oil-in-water emulsion-formulated hair conditioner. The above commercially available hair conditioner without an oil-in-water emulsion was used as a control.

The hair was washed with hair shampoo and then thoroughly rinsed with hot water at around 40°C. Approximately 10g of an oil-in-water emulsion-formulated hair conditioner or a control hair conditioner was taken in the hand, applied well to the entire hair, and left for about 1 minute. Thereafter, the hair was thoroughly rinsed with hot water at around 40°C, the moisture was removed with a towel, and the hair was dried with a dryer. The texture of the hair was then subjected to sensory evaluation by panelists (N = 3).

As a result, when the control hair conditioner was used, the hair became very smooth and glossy, but the hair was less manageable due to electrostatic repulsion, and frizz was observed.

On the other hand, when the oil-in-water emulsion-formulated hair conditioner was used, the hair became very smooth and glossy as in the case of using the control hair conditioner, and furthermore, the hair became moisturized with a moist texture and manageable without frizz or the like due to static electricity. This result suggests that inclusion of the oil-in-water emulsion allows the active component contained in the hair conditioner to be more retained in the hair, and enhances and/ or prolongs the effect of the active component, thereby enhancing moisture retention effect and the like, reducing the surface potential of the hair which affects frizz or the like, and further reducing repulsion between the hair.

As described above, the oil-in-water emulsion according to the present invention is capable of adhering to a predetermined site, to hold the active component, and/or maintain the active component through absorption and adsorption for retention, and enhancing and/or prolonging the effect of the active component. The oil-in-water emulsion is expected to be used as a retention agent for the active component in various fields such as foods or beverages, medicines, and cosmetics.

## Claims

1. A retention agent to be used for retaining an active component, the retention agent comprising an oil-in-water emulsion comprising water, a hydrophobic substance, cyclodextrin, and a thickening polysaccharide.

2. The retention agent according to claim 1, wherein the hydrophobic substance is one or more selected from the group consisting of fat or oil, hydrophobic wax, and hydrophobic resin.

3. The retention agent according to claim 1, wherein the thickening polysaccharide is one or more selected from the group consisting of carboxymethyl cellulose (CMC), xanthan gum, locust bean gum, guar gum, glucomannan, κ-carrageenan, 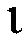 -carrageenan, λ-carrageenan, tamarind gum, gellan gum, gum arabic, pectin, distarch phosphate, hydroxypropyl starch, hydroxypropyl distarch phosphate, tragacanth gum, hydroxypropyl cellulose, methyl cellulose, hydroxypropylmethyl cellulose, and hydroxyethyl cellulose.

4. The retention agent according to claim 1, which is used by being added to a food or beverage.

5. The retention agent according to claim 1, which is used by being added to a medicine.

6. The retention agent according to claim 1, which is used by being added to a cosmetic.

7. A method for producing a retention agent to be used for retaining an active component, the method comprising mixing water, a hydrophobic substance, cyclodextrin, and a thickening polysaccharide to form an oil-in-water emulsion.

8. A food or beverage, comprising a retention agent to be used for retaining an active component, the retention agent comprising an oil-in-water emulsion comprising water, a hydrophobic substance, cyclodextrin, and a thickening polysaccharide, wherein the retention agent is contained in an amount of 0.5 mass% to 55 mass% in terms of an amount of the hydrophobic substance.

9. A medicine, comprising a retention agent to be used for retaining an active component, the retention agent comprising an oil-in-water emulsion comprising water, a hydrophobic substance, cyclodextrin, and a thickening polysaccharide, wherein the retention agent is contained in an amount of 0.5 mass% to 55 mass% in terms of an amount of the hydrophobic substance.

10. A cosmetic, comprising a retention agent to be used for retaining an active component, the retention agent comprising an oil-in-water emulsion comprising water, a hydrophobic substance, cyclodextrin, and a thickening polysaccharide, wherein the retention agent is contained in an amount of 0.5 mass% to 55 mass% in terms of an amount of the hydrophobic substance.

11. Use of an oil-in-water emulsion comprising water, a hydrophobic substance, cyclodextrin and a thickening polysaccharide, in a method for producing a retention agent to be used for retaining an active component.
